Europäisches Patentamt

European Patent Office

Office européen des brevets

(11) Publication number: **0 332 464**
**A2**

# EUROPEAN PATENT APPLICATION

(21) Application number: **89302401.8**

(22) Date of filing: **10.03.89**

(51) Int. Cl.⁴: **A 61 K 37/50**

(30) Priority: **11.03.88 JP 57955/88**

(43) Date of publication of application:
**13.09.89 Bulletin 89/37**

(84) Designated Contracting States: **DE FR GB IT SE**

(71) Applicant: **TOYO JOZO CO., LTD.**
**632-1, Mifuku Ohito-cho**
**Tagata-gun Shizuoka-ken (JP)**

(72) Inventor: **Nishimura, Katsumi**
**149-4, Daiba Mishima-shi**
**Shizuoka 411 (JP)**

(74) Representative: **Sheard, Andrew Gregory et al**
**Kilburn & Strode 30, John Street**
**London WC1N 2DD (GB)**

A request for correction the description has been filed pursuant to Rule 88 EPC. A decision on the request will be taken during the proceedings before the Examining Division (Guidelines for Examination in the EPO, A-V, 2.2).

(54) Superoxide dismutase polypeptides in the prevention of metastasis of malignant tumour cells.

(57) Polypeptides having superoxide dismutase activity can be used as preventive agents for the metastasis of malignant tumour cells. The polypeptides may be natural or muteins. They exhibit no effect of the inhibition of platelet aggregation.

EP 0 332 464 A2

Bundesdruckerei Berlin

## Description

## SUPEROXIDE DISMUTASE POLYPEPTIDES IN THE PREVENTION OF METASTASIS OF MALIGNANT TUMOUR CELLS

This invention relates to a preventive agent for the metastasis of malignant tumor cells, which contains a polypeptide having the activity of superoxide dismutase as the active ingredient.

Abnormal proliferation, invasion to the normal tissues, and metastasis are three characteristics that show cancer is malignant.

Once the metastatic lesions have been formed in cancer patients, its treatment is as difficult as, or even more difficult than, that of the primary lesions. Furthermore, biological characteristics of malignant tumor which affect the prognosis of cancer patients are the invasion to the normal tissues and the metastasis. Metastasis is defined as a phenomenon that malignant tumor cells move from primary lesions to the other regions and form cancer lesions. [Metabolism and Disease, 18, 891-899, Extra-Edition, Gann, 81 (1981)].

However, the treatment of patients with distant metastasis is particularly impossible even with various therapies at the present moment. Most cancer patients die of the growth promotion of metastatic foci, rather than of growth of the primary lesions. Therefore, the prevention of cancer metastasis and its therapy are the most important target for the extermination of cancer.

In the meantime, the process of the metastasis of cancer is generally considered to be formed with the following steps: 1. Release of malignant tumor cells from the primary lesions (and invasion to the circumferential tissues). 2. Intravation of the malignant tumor cells. 3. Dissemination of the malignant tumor cells through the vessels. 4. Lodgement of the malignant tumor cells (arrest by microvessels). 5. Extravasation of the malignant tumor cells. 6. Growth promotion of the malignant tumor cells at the site of metastasis (formation of the metastatic lesions). [Cancer and Chemotherapy, 12, No. 6, 1189-1195, (June 1985); Japanese J. of Clinical Medicine, 44, No. 2, 111-119, (Feb., 1986).]

From the above facts, various basic and clinical studies have been conducted for the means to prevent the metastasis. For example, there are a method for the inhibition of the lodgement of tumor thrombus with anti-coagulants, accelerating agents for fibrinolysis, inhibitors for platelet aggregation, etc., based upon the fact that the coagulability has the relationship with the formation of metastatic lesions (Cancer and Chemotherapy, ibid.; Japanese J. of Clinical Medicine, ibid.; and Pharmaceutical Journal, 22, No. 2, 77-85, 1986); a method for the growth inhibition of micrometastatic lesions by nonspecifically enhancing the immune response of host using various immuno-adjuvants (Metabolism and Disease, ibid.); and a method for systemic chemotherapy and radiotherapy (Metabolism and Disease, ibid.).

However, satisfactory effects have not been obtained with any of these methods.

So far, as the polypeptides having the activity of superoxide dismutase, it is well-known that a polypeptide containing copper and zinc atoms (Cu, Zn-SOD), a polypeptide containing manganese atom (Mn-SOD), and a polypeptide containing iron atom (Fe-SOD), are widely available in animals, plants and microorganisms [Advance in Enzymology, 41, 35-97, (1974)]. As for the activity of this superoxide dismutase, it reacts to superoxide anion radical ($O^-_2$) substrate to produce hydrogen peroxide ($H_2O_2$).

$$2O^-_2 + 2H^+ \rightarrow O_2 + H_2O_2$$

Thus, the polypeptides are metal polypeptides having the activity to catalyze the reaction as shown in the above. Moreover, these polypeptides having the activity of superoxide dismutase are useful for eliminating superoxide anion radicals which are harmful to cells and tissues, and are used, for example, for the treatment of rheumatoid arthritis [Current Therapeutic Research, 20, 62-69, (1976)] and as an anti-inflammatory agent for skin (Japanese Unexamined Patent Publication No. 62-501072).

The polypeptides having the activity of superoxide dismutase do not show anti-tumor activity nor anti-coagulation activity, and no reports have been available on whether or not they act effectively against the metastasis of malignant tumor cells.

The inventor has unexpectedly found out that the polypeptides having the activity of superoxide dismutase were useful as a preventive agent for the metastasis of malignant tumor cells, from the results of the experiment in which they are administered to tumor-bearing experimental animals.

This invention has been based upon the above findings, and it relates to a preventive agent for the metastasis of malignant tumor cells, which contains a polypeptide having the activity of superoxide dismutase as the active ingredient.

As the polypeptides having the activity of superoxide dismutase, being used in this invention, there are no limitation, as far as they have the activity of superoxide dismutase. They may be natural polypeptides obtained by extraction and purification from any natural origin, by chemical synthesis, or by a gene manipulation process, and mutein polypeptides which hold the original activity of superoxide dismutase with a partly converted peptide composition, prepared from natural polypeptides by chemical synthesis or gene manipulation process [Glossary of Genetics and Cytogenetics, 4th Edition, page 381, (1976)].

As the natural polypeptides having the activity of superoxide dismutase according to this invention there are exemplified polypeptides containing copper and zinc atoms, widely distributed in the yeasts, and placenta, liver and blood of higher animals such as rabbit, cattle, horse, sheep, chicken, monkey, human, etc., (Cu, Zn-SOD; a dimer with a molecular weight of about 32,000), polypeptides containing manganese atom, distributed in procaryotes or mitochondoria (Mn-SOD); a dimer with a molecular weight of about 40,000), and polypeptides containing iron atom (Fe-SOD; a dimer with a molecular weight of about 40,000). The preferable polypeptides include a natural polypeptide originated from bovine liver (Olgotein Nonproprietary name adopted by the United States Adopted Name Council; J.A.M.A., Vol. 208, No. 8, May 26, 1969) and a natural polypeptide originated from human (Biochemistry, 19, 2310-2316, 1980, FEBS Letters, 120, 53-55, 1980). Furthermore, to obtain, for example, a human-originated polypeptide, Cu, Zn-SOD, having the activity of superoxide dismutase, which is a dimer consisting of at least 153 amino acids, RNA is collected from human liver tissue, and then, after collecting poly(A) + RNA, c-DNA is synthesized according to the conventional method in gene manipulation, followed by recombining it into an expression vector. Thereafter, it is transformed into a microorganism such as E. coli, yeasts, etc. The microorganism thus obtained is incubated to obtain the objective dimer polypeptide having the activity of superoxide dismutase. The above gene manipulation may be conducted according to any of the methods described in Japanese Kokai 60-137286, 61-111690, 61-139390, 62-115277 and 62-130689 publications. Furthermore, the human polypeptide having the activity of superoxide dismutase according to such a gene manipulation may be a mutein polypeptide which hold the original activity of superoxide dismutase with a partly converted peptide composition. For example, a site-specific variation method may be utilized for the expression vector obtained as stated above. That is, BamH 1 is allowed to react to a closed ring plasmid vector containing a polypeptide DNA having the activity of natural human superoxide dismutase in the presence of ethidium bromide under light shading condition to give its ring-opened structure, followed by adding exonuclease II to prepare a synthetic single strand DNA containing a base sequence which codes the amino acids at the part to be converted. Both of the obtained DNAS are annealed. In the presence of dNTP, DNA polymerase and $T_4$ ligase are added thereto, thereby the synthetic DNA is combined with the plasmid vector, so that an objective vector having a recombined specific site in the gene sequence is obtained [Experimental Manipulation of Gene Expression, 291-303, (1983); Academic Press]. This vector may be transformed into a microorganism by the conventional method of gene manipulation to express the dimer polypeptide. As the means of such a gene manipulation, for example, Japanese Kokai 62-130684 publication can be effectively referred to. For example, as the mutain polypeptides from a Cu, Zn-SOD polypeptide having the activity of natural human superoxide dismutase, which is a dimer consisting of 153 amino acids and possesses N-terminal alanine at the position 1, there are exemplified those in which either one or both of cystein (Cys) at the position 6 and 111 are substituted with other amino acids, particularly, those mutain polypeptides having the activity of superoxide dismutase, with a hydrogen atom or an acetyl group at the N-terminus, in which Cys at the position 111 is substituted with serine (Ser), ([111]Ser-superoxide dismutase peptide: hereinafter occasionally referred to as [111]Ser-SOD), tyrosine (Tyr),

alanine (Ala), phenylalanine (Phe), tryptophan (Trp), arginine (Arg), glysine (Gly), proline (Pro), threonine (Thr), histidine (His), asparagine (Asn), asparaginic acid (Asp), leucine (Leu), valine (Val), glutamine (Gln), glutamic acid (Glu), lysine (Lsy) or methionine (Met). Among these mutein polypeptides having the activity of superoxide dismutase, those in which the Cys at the position 6 remains as it is, or is substituted with a neutral amino acid, such as Ser, Ala, Thr, etc., and the Cys at the position 111 is substituted with a neutral amino acid, such as Ser, Ala, Thr, etc., are preferred.

Such natural polypeptides or mutein polypeptides, having the activity of superoxide dismutase, if necessary, may be combined, directly or through triazine, with 0.5 - 50 parts of a polyalkyleneglycol, such as polyethylene glycol, polypropylene glycol, ethylene oxide-propylene oxide copolymer, etc. with a molecular weight of 400 - 10,000, per molecule of the polypeptide, to produce the polyethylene glycol derivatives (Japanese Kokai 61-249388 and 62-115280 publications). They may be derived to their nontoxic soluble salts, if desired.

Furthermore, these polypeptides having the activity of superoxide dismutase are prepared as injectable preparations, if desired. Simply, freeze-dried injectable preparations of the polypeptides having the activity of superoxide dismutase may be produced as they are, or in combination with a freeze stabilizer, for exmaple, a protein or an amino acid such as albumin, globulin, glutamine, glutamic acid, asparagine, asparaginic acid, glycine, alanine, etc., or a saccharide such as glucose, sucrose, mannitol, sorbitol, maltose, etc. In this case, the concentration of the poly peptide having the activity of superoxide dismutase in the preparation may be adjusted so as to be 0.5 - 100 mg/ml with the administration amount of 0.1 - 2 ml, 1 - 5 times daily. The preparation may be adjusted by the addition of an osmotic pressure regulator, preservative or pH adjuster, if necessary, and is usually administered in an amount of 1 -250 mg, preferably 5 - 100 mg, to an adult. Moreover, the polypeptide having the activity of superoxide dismutase may be prepared to form each 1 - 2 g amount of suppository preparation, using an oily base for suppository, such as oils and fats, i.e., olive oil, peanut oil, sesame oil, soybean oil, rapeseed oil, camellia oil, cacao fat, lard, lanolin, beef tallow, etc., their hydrogenated oil, their acetylated compounds, with a single or two or more emulsified substances obtained from a surfactant, fatty acid salt, and an absorption accelerator such as N-acylamino acid salts, etc.

The preparations of the polypeptides having the activity of superoxide dismutase thus obtained demonstrate significant effects on the prevention of metastasis in an experimental system of metastatic model using Lewis lung-carcinoma and Meth-A fibrosarcoma, with no effect on the inhibition of the platelet aggregation as shown in the following exmaples. Thus, they are useful in the effectively prevention of the metastasis of the cells of malignant epithelial tumors,such as gastric cancer, lung cancer, large intestinal cancer, breast cancer, prostate cancer, utero-cervical cancer, renal cancer, etc., as well as the cells of the other malignant non-epithelial tumors.

This invention is explained in more detail with respect to the examples, but they are not to be construed as the limitations cf this invention.

EXAMPLE 1

Experiment 1

Lewis lungcarcinoma continuous transplanted in the subcutis of BDF 1 mice were aseptically extirpated, treated with 0.1 % of collagenase for 2 hours, and then filtered using a mesh of 250. Further, they were collected by centrifuge with 3000 rpm for 5 minutes, and the number of the cells was adjusted to $2 \times 10^6$/ml in a Fischer's medium containing 10 % of bovine serum.

The polypeptide (called SOD-1) having the activity of Cu, Zn-type superoxide dismutase originated from human in which [111]Cys has been site-specifically substituted with Ser, was obtained by incubation of an E. col DH1pSOD14 (FERM BP-1696) strain having plasmid pSOD14 prepared by the method mentioned in Japanese Kokai 62-130684 publication. It was dissolved in distilled water to make a preparation for injection.

An amount of 0.25 ml of $2 \times 10^6$/ml of the above mentioned Lewis lungcarcinoma cells was inoculated subcutaneously to BDF1 mice as the subject animal (4-weeks old, male, 1 group: 7). Each 10 mg/Kg of SOD-1 (SOD Injection: 0.1 ml/10 g of body weight of mouse) was administered subcutaneously from the following day for 7 days (hereinafter, called A group). As the B group, 10 mg/Kg of SOD-1 (SOD Injection 0.1 ml/10 g of body weight of mouse) was administered from the 8th to 14th days after the subcutaneous injection of Lewis lungcarcinoma cells.

As the control group, no SOD-1 was administered after the subcutaneous injection of Lewis lungcarcinoma cells.

The lungs of all the groups were extirpated on the 21st day after the subcutaneous injection of Lewis lungcarcinoma cells, and the number of metastatic foci on the lung surface was calculated using a stereoscopic microscope. The results are shown in Table 1.

Table 1

| | No. of metastatic foci |
|---|---|
| Control | $48.2 \pm 12.3$ |
| A group | $24.5 \pm 4.4$ ** |
| B group | $19.0 \pm 7.2$ ** |

**) T-test: 1 % significant different

From the above results, it is demonstrated that the number of pulmonary metastatic foci was significantly inhibited in the groups to which the polypeptide having the activity of superoxide dismutase had been administered. However, no differences were observed regarding tumor growth at the transplantation site in comparison with the control group. Since the better effect was observed in B group with the administration for the 8th - 14th days, than in A group with the 1st - 7th days, it is presumed that the release or metastasis of malignant tumor cells from the primary lesion begins at this stage.

Experiment 2

Meth-A fibrosarcoma continuous subcultured in the ascites of Balb/c mice was aseptically collected, treated with a 0.83 % ammonium chloride solution, and washed twice with a Fischer's medium, and the number of the cells was adjusted to $2.5 \times 10^6$/ml.

An amount of 10 mg/Kg of SOD-1 (SOD Injection: 0.1 ml/10 g of body weight of mouse) was administered intravenously to Balb/c mice as the subject animal (4-weeks old, male, 1 group: 7), and 5 minutes later, 0.20 ml of $2.5 \times 10^6$/ml of the aforementioned Meth-A fibrosarcoma cells was inoculated intravenously (hereinafter, called A group). As the control groups, a control group 1 without SOD-1 and a control group 2 with the administration of the same amount of a physiological saline solution, instead of SOD-1, were prepared. SOD-1 and tumor cells were injected from the same site as a rule, and only in a difficult case, injected from the nearer site to the root of tail than previously injected site.

The lungs of all the groups were extirpated on the 14th day after the inoculation or Meth-A fibrosarcoma, and the number of metastatic foci on the lung surface was calculated using a stereoscopic microscope. The results are shown in Table 2.

Table 2

| | No. of metastatic foci |
|---|---|
| Control 1 | $178.4 \pm 67.3$ |
| Control 2 | $154.8 \pm 45.3$ |
| A group | $36.4 \pm 26.7$ ** |

**) T-test: 1 % significant different

Experiment 3

Instead of the dose in SOD-1, i.e., 1 mg/Kg, in the said experiment 2, doses of 10 mg/Kg and 50 mg/Kg were administered respectively to Balb/c mice (4 weeks old, male, 1 group: 7), and the experiments were conducted in the same way as the experiment 2.

As the results, a subject which had a less number of metastatic foci was found in the group of 1 mg/Kg, but no significant difference was observed in comparison with the control group, if it was exempted. Moreover, significant differences of the number of metastatic foci were observed in the groups of 10 mg/Kg and 50 mg/Kg, when compared with the control group.

Experiment 4

Instead of the said SOD-1, a polypeptide having Cu, Zn-type superoxide dismutase originated from human erythrocyte (hereinafter called SOD-2) was employed, and the preventive effect on metastatic foci of malignant tumor cells was studied.

Cells of Meth-A fibrosarcoma continuous subcultured in the ascites of Balb/c mice were aseptically collected, treated with a 0.83 % ammonium chloride solution, and washed twice with a Fischer's medium. The number of the cells was adjusted to $2.5 \times 10^6$/ml.

An amount of 0.2 mg/Kg, 1 mg/Kg, 5 mg/Kg or 10 mg/Kg, of SOD-2 (SOD Injection: 0.1 ml/10 g of body weight of mouse) was administered intravenously to Balb/c mice (5 weeks old, male, 1 group: 7) respectively, and 15 minutes later, 0.20 ml of $2.5 \times 10^6$/ml of the aforementioned Meth-A fibrosarcoma cells was inoculated intravenously (hereinafter called A group). As the control groups, a control group 1 without SOD-2 and a control group 2 with the administration of the same dose of a physiological saline solution, instead of SOD-2, were prepared. SOD-2 and tumor cells were injected from the same site as a rule, and only in a difficult case, injected from the nearer site from the root of tail than previously injected site.

The lungs of all the groups were extirpated on the 14th day after the inoculation of Meth-A fibrosarcoma, and the number of metastatic foci was calculated using a stereoscopic microscope. The results are shown in Table 3.

Table 3

|  | No. of metastatic foci |
|---|---|
| Control 1 | 154.8 ± 71.7 |
| Control 2 | 122.7 ± 27.8 |
| A group |  |
| 0.2 mg | 45.5 ± 27.6 * |
| 1 | 68.6 ± 24.3 |
| 5 | 33.8 ± 25.9 * |
| 10 | 25.8 ± 8.6 * |

*) T-test: 5% significant different (compared with control 1)

From the above results, it was proved that SOD-2 significantly prevented the metastasis of malignant tumor cells, also.

Reference Example 1

Studies on the inhibitory effect of the polypeptide having the activity of superoxide dismutase against the platelet aggregation caused by malignant tumor cells, using Platelet Rich Plasma of rabbits (hereinafter, abbreviated PRP).

Both of Meth-A fibrosarcoma and Lewis lungcarcinoma increase the platelet aggregation dependent on the number of the cells, but under the condition of PRP added with sodium citrate, no aggregation is induced without the addition of 5 mM of calcium chloride.

Accordingly, studies on whether or not the polypeptide having the activity of superoxide dismutase inhibits the aggregation were conducted under aggregating conditions of Assays I and II as mentioned below. As the polypeptide having the activity of superoxide dismutase, the SOD-1 and SOD-2 were used in concentrations of 1 mg/ml, 5 mg/ml and 10 mg/ml, respectively.

Assay I

| PRP added with 0.38 sodium citrate | 0.3 ml |
|---|---|
| Malignant tumor cells ($1 \times 10^5$/10 $\mu\ell$) | 10 $\mu\ell$ |
| Calcium chloride (5 mM, final concentration) | 10 $\mu\ell$ |
| SOD-1 or SOD-2 | 10 $\mu\ell$ |

Assay II

| PRP (heparin: 4 units/ml blood) | 0.3 ml |
|---|---|
| Malignant tumor cells ($1 \times 10^5$/10 $\mu\ell$) | 10 $\mu\ell$ |
| SOD-1 or SOD-2 | 10 $\mu\ell$ |

However, the inhibition of platelet aggregation caused by Meth-A fibrosarcoma cells or Lewis lungcarcinoma cells was not found in any concentration of SOD-1 or SOD-2.

It was judged from these results that the polypeptide having the activity of superoxide dismutase exhibits no effect to inhibit the platelet aggregation.

As mentioned above, the present invention is based upon the findings that the polypeptides having the activity of superoxide dismutase which exhibit no effect of the inhibition of platelet aggregation, significantly prevent the metastasis of malignant tumor, in a way completely different from anti-coagulants, accelerating agents for fibrinolysis, inhibitors for platelet aggregation, etc. which have been used for the prevention of the metastasis of malignant tumor. Moreover, the administration in a large dose is possible, since the polypeptides having the activity of superoxide dismutase themselves are less toxic. Thus, the present invention can provide for useful preventive agents for the metastasis of malignant tumor cells, even in their prophylaxis use, and for the preventive methods of the metastasis of malignant tumor cells, based upon them.

**Claims**

1. The use of a polypeptide having superoxide dismutase activity in the preparation of a preventive agent for the metastasis of malignant tumour cells.

2. The use as claimed in Claim 1, wherein the polypeptide is a natural polypeptide.

3. The use as claimed in Claim 1, wherein the polypeptide is a mutein polypeptide.

4. The use as claimed in Claim 3, wherein the mutein polypeptide is [111]Ser-superoxide dismutase polypeptide.

5. A pharmaceutical composition comprising a polypeptide having superoxide dismutase activity in an amount effective to prevent metastasis of malignant tumour cells and a pharmaceutically acceptable carrier.

6. A composition as claimed in Claim 5, in which the carrier comprises a freeze stabiliser.

7. A composition as claimed in Claim 5, wherein the carrier comprises a suppository base.

8. A sterile injectable preparation of a polypeptide having superoxide dismutase activity.